(19) Europäisches Patentamt
European Patent Office
Office européen des brevets

(11) Veröffentlichungsnummer : **0 014 390**
**B1**

(12) **EUROPÄISCHE PATENTSCHRIFT**

(45) Veröffentlichungstag der Patentschrift : **03.02.82**

(21) Anmeldenummer : **80100363.3**

(22) Anmeldetag : **24.01.80**

(51) Int. Cl.³ : **C 07 D471/04**, A 23 K 1/16, A 61 K 31/505 // (C07D471/04, 221/00, 239/00)

(54) **2-Amino-8-cyclopropyl-5-oxo-5,8-dihydro-pyrido (2,3-d) pyrimidin-6-carbonsäuren, sie enthaltende Arzneimittel sowie Verfahren zu ihrer Herstellung.**

(30) Priorität : **01.02.79 DE 2903850**

(43) Veröffentlichungstag der Anmeldung : **20.08.80 (Patentblatt 80/17)**

(45) Bekanntmachung des Hinweises auf die Patenterteilung : **03.02.82 Patentblatt 82/05**

(84) Benannte Vertragsstaaten : **AT BE CH DE FR GB IT NL SE**

(56) Entgegenhaltungen :
**DE - A1 - 2 733 194**
**DE - B2 - 2 143 369**
**DE - B2 - 1 670 533**
**GB - A - 1 451 911**
**GB - A - 1 451 912**
**GB - A - 1 530 413**

(73) Patentinhaber : **BAYER AG**
**Zentralbereich Patente, Marken und Lizenzen**
**D-5090 Leverkusen 1, Bayerwerk (DE)**

(72) Erfinder : **Grohe, Klaus, Dr.**
**Am Wasserturm 10**
**D-5068 Odenthal (DE)**
Erfinder : **Zeiler, Hans-Joachim, Dr.**
**Windratherstrasse 188**
**D-5620 Velbert 15 (DE)**
Erfinder : **Metzger, Karl Georg, Dr.**
**Pahlkestrasse 15**
**D-5600 Wuppertal 1 (DE)**

**EP 0 014 390 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# 2-Amino-8-cyclopropyl-5-oxo-5,8-dihydro-pyrido [2,3-d] pyrimidin-6-carbonsäuren, sie enthaltende Arzneimittel sowie Verfahren zu ihrer Herstellung

Die vorliegende Erfindung betrifft neue 2-Amino-8-cyclopropyl-5-oxo-5,8-dihydropyrido [2,3-d] pyrimidin-6-carbonsäuren, Verfahren zu ihrer Herstellung sowie ihre Verwendung zur Herstellung von Arzneimitteln, insbesondere antibakterielle Mittel, und Futterzusatzmittel.

Es ist bereits bekannt geworden, daß 2-Amino-8-äthyl-5-oxo-5,8-dihydro-pyrido [2,3-d] pyrimidin-6-carbonsäuren antibakterielle Eigenschaften besitzen [Eur. J. Med. Chem. 9, 591-596 (1974)].

Es wurde nun gefunden, daß die neuen 2-Amino-8-cyclopropyl-5-oxo-5,8-dihydropyrido [2,3-d] pyrimidin-6-carbonsäuren der Formel I.

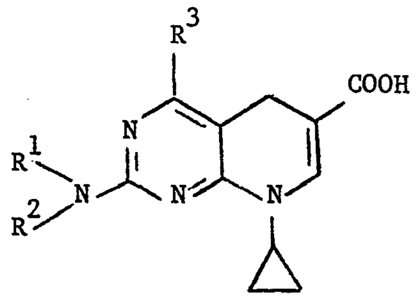

(I)

in welcher

$R^1$ und $R^2$ gleich oder verschieden sein können und für Wasserstoff, einen verzweigten oder unverzweigten Alkylrest mit 1-12 Kohlenstoffatomen, einen Alkenyl- oder Alkinylrest mit 3-12 Kohlenstoffatomen stehen, wobei sich die Doppel- bzw. Dreifachbindung nicht an dem mit dem Stickstoffatom verbundenen Kohlenstoffatom befinden darf, und der gegebenenfalls durch Hydroxylgruppen, Alkoxy-, Alkylmercapto- oder Dialkylamino-gruppen mit 1-3 Kohlenstoffatomen für einen Alkylrest, die Nitril-, Alkoxycarbonyl-gruppe mit 1-4 Kohlenstoffatomen im Alkoxyteil sowie einen Aryl- oder Hetaryl-rest substituiert sein kann, ferner Cycloalkyl mit 3-6 Kohlenstoffatomen bedeutet und weiterhin gemeinsam mit dem Stickstoffatom, das sie substituieren und gegebenenfalls Sauerstoff, Schwefel oder $NR^4$ einen 3-7-gliedrigen Ring bilden, der ein- oder mehrfach durch einen Alkyl-rest mit 1-6 Kohlenstoffatomen oder Alkenyl-gruppen mit 3-6 Kohlenstoffatomen, wobei sich die Doppelbindung nicht an dem mit dem Stickstoffatom verbundenen Kohlenstoffatom befinden darf, Hydroxyl-, Alkoxy- und Alkylmercaptogruppen mit 1-3 Kohlenstoffatomen, eine Alkoxycarbonylgruppe mit 1-4 Kohlenstoffatomen im Alkoxyteil, die Nitrilgruppe sowie einen Arylrest substituiert sein kann und ferner eine Doppelbindung besitzen kann,

$R^3$ für Wasserstoff oder Alkyl mit bis zu 3 Kohlenstoffatomen steht und

$R^4$ Wasserstoff, eine verzweigte oder unverzweigte Alkyl-, Alkenyl- oder Alkinyl-gruppe mit 1-6 Kohlenstoffatomen, die gegebenenfalls durch Hydroxyl, Alkoxy-Alkylmercapto- und Dialkylaminogruppe mit 1-3 Kohlenstoffatomen für einen Alkylrest, die Alkoxycarbonylgruppe mit 1-4 Kohlenstoffatomen im Alkoxyteil substituiert sein kann, eine Cycloalkylgruppe mit 3-7 Kohlenstoffatomen, eine gegebenenfalls im Arylrest substituierte Aralkylgruppe mit bis zu 4 Kohlenstoffatomen im aliphatischen Teil, sowie eine gegebenenfalls durch Halogen, Alkyl, Alkoxy oder Alkylmercaptogruppen mit 1-3 Kohlenstoffatomen, eine Trifluormethyl-, Nitro- oder Nitrilgruppe substituierte Phenyl- oder Naphtylgruppe oder ein heterocyclischer Rest wie beispielsweise einen Pyridin-, Pyrimidin-, Thiazol- oder Benzthiazolkern darstellt, ferner eine gegebenenfalls durch einen Arylrest substituierte Alkoxycarbonylgruppe mit 1-4 Kohlenstoffatomen im Alkoholteil, ein Alkanoylrest mit 1-6 Kohlenstoffatomen, ein Aroylrest, ein Alkylthiocarbamoylrest mit 1-4 Kohlenstoffatomen im Alkylteil oder ein Arylthiocarbamoylrest oder ein gegebenenfalls durch Halogen, Alkyl, Alkoxy oder Alkylmercaptogruppen mit 1-3 Kohlenstoffatomen, eine Trifluormethyl-, Nitro- oder Nitrilgruppe substituierter Aryl-(thio) carbamoylrest, ein Alkyl- oder Arylsulfonylrest sowie ein gegebenenfalls durch Alkyl substituierter Aminosulfonylrest bedeutet, und deren pharmazeutisch verwendbaren Salze eine den bekannten Pyrido [2,3-d] pyrimidin-6-carbonsäuren überlegene antibakterielle Wirkung aufweisen.

Der obengenannte Arylrest, vorzugsweise der Phenyl- oder Naphthylrest, kann durch Halogen, vorzugsweise Fluor, Chlor und/oder Brom, Alkyl-, Alkoxy- oder Alkylmercaptogruppen mit 1-3 Kohlenstoffatomen, die Aryloxy-, Arylmercapto- sowie eine Trifluormethyl-, Nitro-, Nitril- oder Alkoxycarbonylgruppe mit 1-4 Kohlenstoffatomen im Alkoxyteil ein- oder mehrfach substituiert sein.

Weiterhin wurde gefunden, daß man 2-Amino-8-cyclopropyl-5-oxo-5,8-dihydropyrido [2,3-d] pyrimidin-6-carbonsäuren der Formel I erhält wenn man Pyrido [2,3-d] pyrimidin-6-carbonsäuren der Formel II (R = H)

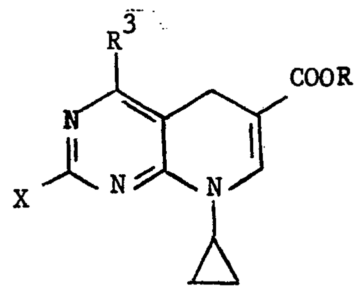

(II)

in welcher

$R^3$ die oben angegebene Bedeutung hat und X für ein Halogenatom oder eine Alkylmercapto- oder Alkoxygruppe mit 1-4 Kohlenstoffatomen steht, mit Aminen der Formel III

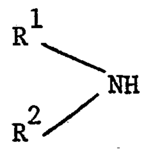

(III)

in welcher

$R^1$ und $R^2$ die oben angegebene Bedeutung haben, umsetzt. Ferner kann man 2-Halogen-pyrido [2,3-d] pyrimidin-6-carbonsäureester II (R = Alkyl) mit III, in Gegenwart eines Säurebinders, wie z.B. Triäthylamin oder Pyridin, umsetzen und anschließend die erhaltenen 2-Amino-pyrido [2,3-d] pyrimidin-6-carbonsäureester alkalisch zu I verseifen.

Der III entsprechende Aminrest kann auch zu einem früheren Zeitpunkt in die 2-Stellung des Pyrimidinkerns eingeführt werden. So erhält man bei der Umsetzung von VIII (X = Halogen) mit III unter schärferen Reaktionsbedingungen [über die Reaktionsbedingungen beim stufenweisen Austausch der beiden Chloratome von 2,4-Dichlor-6-methyl-pyrimidin-5-carbonsäureestern gegen Aminreste vgl. Liebigs Ann. Chem. *1973,* 1025-1035] die 2,4-Diamino-pyrimidincarbonester der Formel XI. Anschließende Dieckmann-Cyclisierung, Dehydrierung, z.B. mit Brom-Triäthylamin, und Verseifung führt dann zu den 2-Amino-pyrido [2,3-d] pyrimidin-6-carbonsäuren der Formel I.

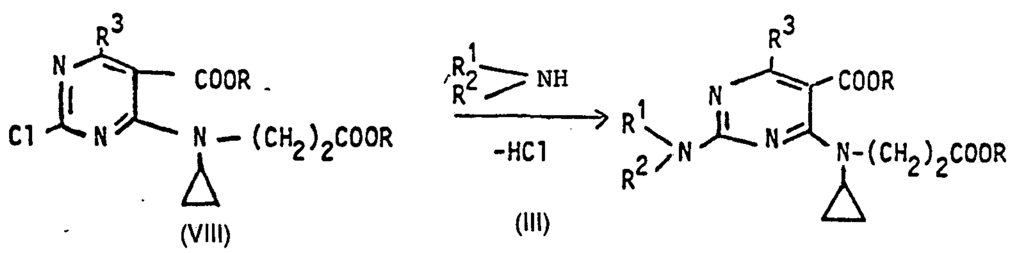


(VIII) (III) (XI)

Verwendet man bei der Umsetzung von II mit III beispielhaft A-Äthylmercapto-8-cyclopropyl-5-oxo-5,8-dihydro-pyrido [2,3-d] pyrimidin-6-carbonsäure und N-Methylpiperazin als Reaktanten, so kann der Reaktionsablauf durch das folgende Formelschema wiedergegeben werden :

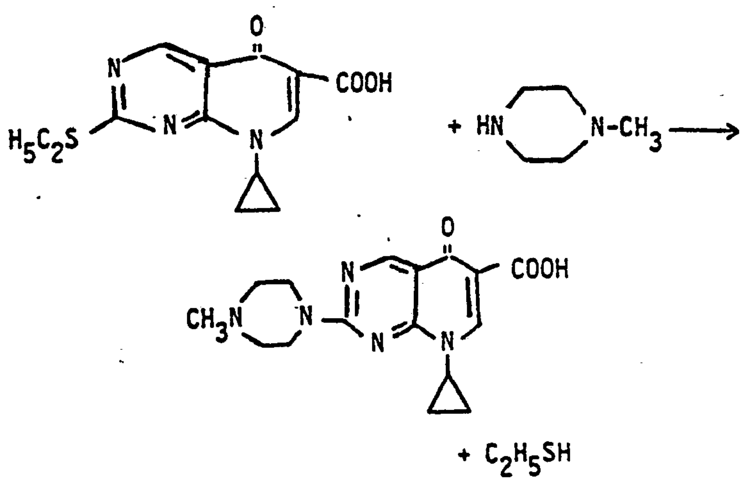

Die Ausgangsverbindungen II können nach verschiedenen Verfahren hergestellt werden :

1. Gemäß einem eigenen älteren Vorschlag (DE-OS-2808070) werden in 2-Stellung substituierte 4-Chlor-pyrimidin-5-carbonsäurechloride der Formel IV mit β-Cyclopropylamino-acrylsäureestern der Formel V zu den, den Carbonsäuren der Formel II entsprechenden Estern cycloaracyliert. Ihre alkalische Verseifung liefert die gewünschten Carbonsäuren der Formel II.

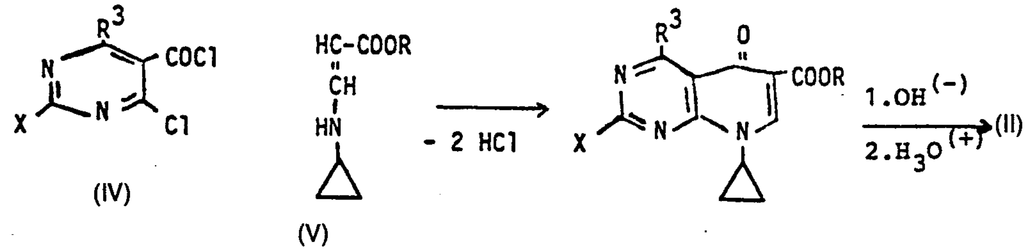

(IV) (V) (II)

Nach dieser Methode können bevorzugt 2-Alkylmercapto-8-cyclopropyl-5-oxo-pyrido [2,3-d] pyrimidin-6-carbonsäuren hergestellt werden.

2. Das zweite Verfahren geht von in 2-Stellung durch X substituierten 4-Halogen-pyrimidin-5-carbonsäureestern der Formel VI aus, die mit β-Cyclopropylamino-propionsäureestern der Formel VII, bevorzugt Methyl- oder Äthylestern, die durch Umsetzung von entsprechenden Acrylsäureestern mit Cyclopropylamin leicht zugänglich sind, weitgehend selektiv unter Austausch des 4-ständigen Halogenatoms gegen den Aminrest zum Monosubstitutionsprodukt der Formel VIII reagieren. Letztere gehen in Gegenwart einer starken Base, wie z.B. Kalium-t-butanolat oder Natriumhydrid, durch

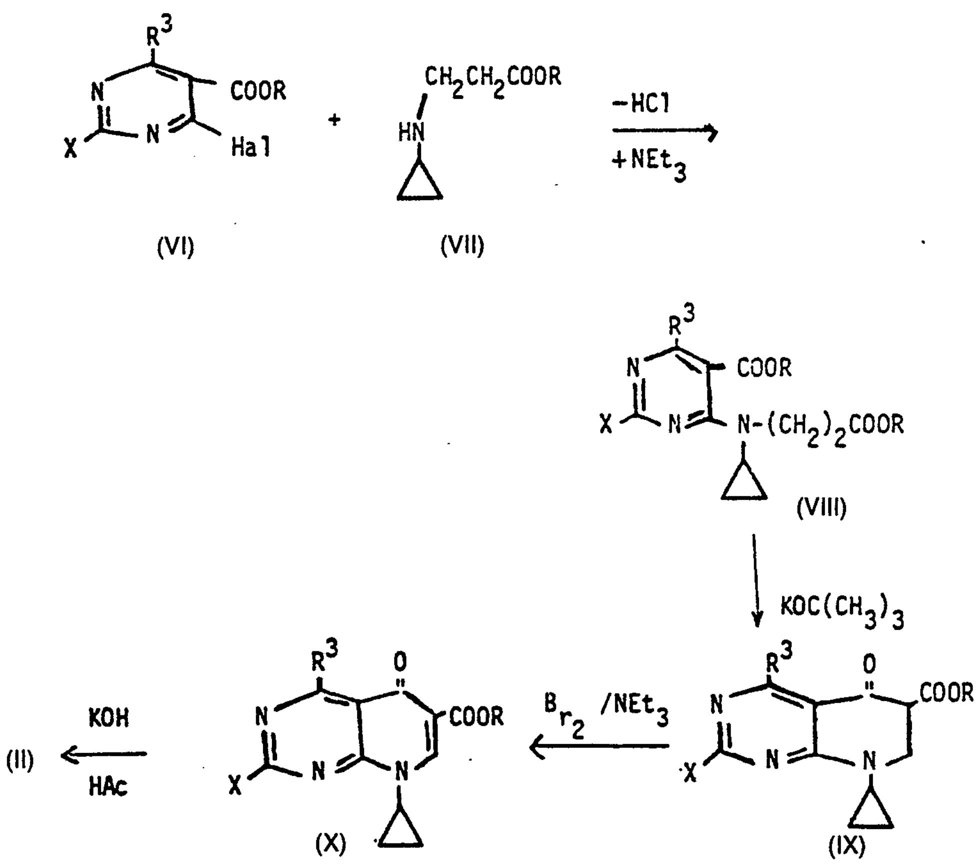

(VI) (VII) (VIII) (II) (X) (IX)

Dieckmann-Cyclisierung in die Dihydro-pyrido [2,3-d] pyrimidin-6-carbonsäureester der Formel IX über. Mit Brom oder Sulfurylchlorid und Triäthylamin oder Pyridin als Dehydrohalogenierungsmittel erhält man aus IX die Carbonsäureester der Formel X, die mit Alkali zu den Carbonsäuren der Formel II (R = H) verseift werden können.

Stellt der Subsituent X der Verbindung X dagegen ein Halogenatom dar, so wird X mit dem Amin III bevorzugt in den I entsprechenden Carbonsäureester übergeführt, der anschließend alkalisch zu I verseift wird.

Die Dihydrocarbonsäureester der Formel IX mit $R^3 = CH_3$ können nur mit Chloranil oder Schwefel unter den üblichen Reaktionsbedingungen zu X dehydriert werden. Durch Brom bzw. Sulfurylchlorid wird in diesem Fall bevorzugt die Methylgruppe in 4-Stellung angegriffen.

Als Verdünnungsmittel kommen inerte organische Lösungsmittel in Frage. Hierzu gehören vorzugsweise Toluol, Dioxan, Dimethylformamid, Dimethylsulfoxid und Sulfolan. Verwendet man anstelle der 2-Alkylmercapto- die entsprechenden 2-Halogen-pyrido [2,3-d] pyrimidin-6-carbonsäureester, so wird vorzugsweise ein Säurebinder zugesetzt. Als solche können vorzugsweise Alkalicarbonate, Alkalihydroxide, tert. organische Basen wie z.B. Triäthylamin, Pyridin usw. dienen.

Die Reaktionstemperaturen können in einem größeren Bereich variieren. Im allgemeinen arbeitet man zwischen etwa 20° und etwa 180 °C, vorzugsweise zwischen 60° und 140 °C.

Die Umsetzung kann bei Normaldruck, aber auch bei erhöhtem Druck, insbesondere bei gasförmigen und niedrig siedenden Aminen der Formel III durchgeführt werden. Im allgemeinen arbeitet man bei Drucken zwischen etwa 1 und etwa 100 bar, vorzugsweise zwischen 1 und 10 bar.

Bei der Durchführung des Verfahrens setzt man auf 1 Mol Carbonsäure 1-4 Mol Amin, vorzugsweise 1,5-2,5 Mol Amin ein. Als neue antibakterielle Wirkstoffe seien im einzelenen genannt:

2-Methylamino-, 2-Dimethylamino-, 2-n-Hexylamino-, 2-Benzylamino-, 2-(4-Methoxybenzyl-amino)-, 2-Pyrrolidino-, 2-Morpholino-, 2-Piperidino-, 2-Hexamethylenimino-, 2-Piperazino-, 2-(4-Methylpiperazino)-, 2-(4-Benzylpiperazino)-, 2-(4-β-Hydroxyäthylpiperazino)-, 2-(4-p-Methoxybenzylpiperazino)-, 2-Pyrrolidino-4-methyl-, 2-Morpholino-4-methyl-8-cyclopropyl-5-oxo-5,8-dihydro-pyrido [2,3-d] pyrimidin-6-carbonsäure und pharmazeutisch verträgliche Säureadditionssalze oder Alkalisalze dieser Verbindungen.

## Beispiel 1

2-(4-Methyl-piperazino)-8-cyclopropyl-5-oxo-5,8-dihydro-pyrido-[2,3-d] pyrimidin-6-carbonsäure (I, $R^1$ $R^2$ N = 4-Methylpiperazino, $R^3$ = H).

Eine Lösung von 2,91 g 2-Äthylmercapto-8-cyclopropyl-5-oxo-5,8-dihydro-pyrido [2,3-d] pyrimidin-6-carbonsäure und 2,2 g N-Methylpiperazin in 30 ml N-Dimethylformamid wird 2,5 Stunden auf 110° erhitzt. Man destilliert das Lösungsmittel im Vakuum ab und kristallisiert den Rückstand aus Äthanol um. Man erhält 2,4 g (73 % der theoretischen Ausbeute) 2-(4-Methyl-piperazino-8-cyclopropyl-5-oxo-5,8-dihydro-pyrido [2,3-d] pyrimidin-6-carbonsäure vom Schmelzpunkt 209-210 °C.

## Beispiele 2 bis 54

Analog der Arbeitsweise im Beispiel 1 wurden die Carbonsäuren der Beispiele 2 bis 54 erhalten. Sie sind in der Tabelle 1 zusammengestellt. Die Bezifferung der Reste $R^1$, $R^2$ und $R^3$ bezieht sich auf die Formel I der Beschreibung.

Tabelle 1

| Beispiel Nr. | $R^1$ $R^2$ | $R^3$ | Zersp. (°C) |
|---|---|---|---|
| 2 | $-CH_2CH_2CH_2CH_2-$ | $CH_3$ | 253 |
| 3 | $-(CH_2)_2O(CH_2)_2-$ | $CH_3$ | 270 |
| 4 | $-CH_2CH_2CH_2CH_2-$ | H | 306 |
| 5 | $-(CH_2)_2O(CH_2)_2-$ | H | 254 |
| 6 | $-CH_2(CH_2)_3CH_2-$ | H | 252 |
| 7 | $-(CH_2)_2-N-(CH_2)_2-$<br>H | H | 246 |
| 8 | $-(CH_2)_2-N-(CH_2)_2-$<br>$(CH_2)_2OH$ | H | 178(+) |
| 9 | 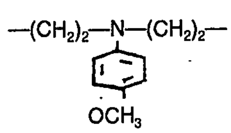 | H | 232 |
| 10 | $-(CH_2)_2-N-(CH_2)_2-$<br>$COOC_2H_5$ | H | 250 |

Tabelle 1 (Fortsetzung)

| Beispiel Nr. | $R^1$ | $R^2$ | $R^3$ | Zersp. (°C) |
|---|---|---|---|---|
| 11 | $CH_3$ | $CH_3$ | H | 228[(++)] |
| 12 | $-(CH_2)_2-N-(CH_2)_2-$<br>$SO_2N(CH_3)_2$ | | H | 302 |
| 13 | $-(CH_2)_2-CH-(CH_2)_2-$<br>$C_2H_5$ | | H | 207 |
| 14 | $-CH_2CH_2(CH_2)_2CH_2CH_2-$ | | H | 227 |
| 15 | $CH_3$ $CH_3$<br>$-CH_2-C-C-CH_2-$<br>$CH_3$ $CH_3$ | | H | 277 |
| 16 | $CH_3$<br>$-CH_2-C-(CH_2)_2-$<br>$CH_3$ | | H | 267 |
| 17 | $CH_3$<br>$-CH_2-C-(CH_2)_3-$<br>$CH_3$ | | H | 170 |
| 18 | $CH_3$<br>$-(CH_2)_2-CH-(CH_2)_2-$ | | H | 212 |
| 19 | $-(CH_2)_2-N-(CH_2)_2-$<br>$(CH_2)_2C_6H_5$ | | H | 229 |
| 20 | $-(CH_2)_2CH=CH-CH_2-$ | | H | 245 |
| 21 | $-(CH_2)_2C(CH_3)=CH-CH_2-$ | | H | 197 |
| 22 | $CH_3$<br>$-(CH_2)_2-C-(CH_2)_2-$<br>$CH_3$ | | H | 276 |
| 23 | $-(CH_2)_2-N-(CH_2)_2-$<br>$CH_2-C_6H_5$ | | H | 226 |
| 24 | $-(CH_2)_2-N-(CH_2)_2-$<br>$O=C-CH(CH_3)_2$ | | H | 228 |
| 25 | $-(CH_2)_2-C=CH-CH_2-$<br>$n-C_4H_9$ | | H | 173 |
| 26 | $-(CH_2)_2-N-(CH_2)_2-$<br>$C_6H_5$ | | H | 252 |
| 27 | $-(CH_2)_2-N-(CH_2)_2-$<br>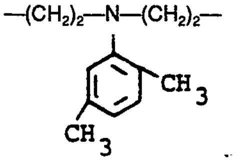 | | H | 217 |
| 28 | $-(CH_2)_2-N-(CH_2)_2-$<br>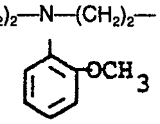 | | H | 265 |

Tabelle 1 (Fortsetzung)

| Beispiel Nr. | $R^1$ | $R^2$ | $R^3$ | Zersp. (°C) |
|---|---|---|---|---|
| 29 | $-(CH_2)_2-N-(CH_2)_2-$<br>CHO | | H | 285 |
| 30 | $-(CH_2)_2-N-(CH_2)_2-$<br>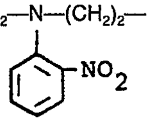 | | H | 232 |
| 31 | H | $CH_3$ | H | 282(++) |
| 32 | $-(CH_2)_2-N-(CH_2)_2-$<br>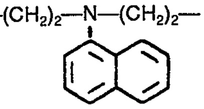 | | H | 262 |
| 33 | $-(CH_2)_2-N-(CH_2)_2-$<br>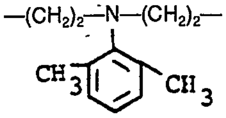 | | H | 218 |
| 34 | $-(CH_2)_2-N-(CH_2)_2-$<br>$COOCH_2C_6H_5$ | | H | 235 |
| 35 | $CH_3$ $CH_3$<br>$-CH_2-CH-N-CH-CH_2-$<br>$C_2H_5$ | | H | 142 |
| 36 | $-(CH_2)_3-CH-CH_2-$<br>OH | | H | 287 |
| 37 | $-(CH_2)_2-CH-(CH_2)_2-$<br>OH | | H | 215 |
| 38 | $-(CH_2)_2-N-(CH_2)_2-$<br>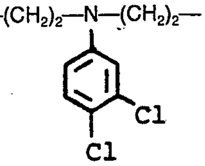 | | H | 232 |
| 39 | $-(CH_2)_2-N-(CH_2)_2-$<br>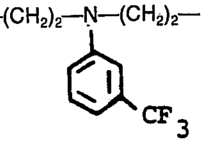 | | H | 237 |
| 40 | $-(CH_2)_2-N-(CH_2)_2-$<br>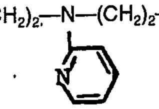 | | H | 314 |
| 41 | $CH_3(CH_2)_5-$ | H | H | 177 |
| 42 | $CH_3$ | $-CH_2CH_2OH$ | H | 194 |
| 43 | $H_2C=CH-CH_2-$ | H | H | 265 |
| 44 | $CH_3(CH_2)_{11}-$ | H | H | 187 |
| 45 | $-(CH_2)_2-C=C-CH_2-$<br>$CH_3$ $CH_3$ | | H | 247 |
| 46 | $(C_2H_5)_2N-CH_2CH_2-$ | H | H | 178 |
| 47 | $CH_3OCH_2CH_2-$ | H | H | 265 |

Tabelle 1 (Fortsetzung)

| Beispiel Nr. | $R^1$ $R^2$ | $R^3$ | Zersp. (°C) |
|---|---|---|---|
| 48 | $-CH_2-CH_2-S-CH_2-$ | H | 284 |
| 49 | $-CH_2CH-(CH_2)_3$<br>$COOC_2H_5$ | H | 188 |
| 50 | $-(CH_2)_2-N-(CH_2)_2-$<br>$(CH_2)_3OH$ | H | 190 |
| 51 | $-(CH_2)_2-CH-(CH_2)_2$<br>$COOC_2H_5$ | H | 222 |
| 52 | $-(CH_2)_2-N-(CH_2)_2-$<br>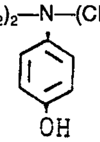 | H | 299 |
| 53 | $-(CH_2)_2-N-(CH_2)_2-$<br>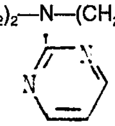 | H | 342 |
| 54 | $-(CH_2)_2-S-(CH_2)_2$ | H | 309 |

($^+$) Wurde als N-Hydroxyäthyl-piperazinsalz isoliert.
($^{++}$) Das gasförmige Amin wurde 3 Stunden bei 110 °C in die Lösung der 2-Alkylmerkapto-pyrido [2,3-d] pyrimidin-carbonsäure eingeleitet.

Die als Ausgangsmaterial verwendeten 2-Alkylmercapto-8-cyclopropyl-5-oxo-5,8-dihydro-pyrido [2,3-d] pyrimidin-6-carbonsäuren können auf zwei verschiedenen Wegen synthetisiert werden :

A. Nach dem Verfahren der oben erwähnten DE-OS-2808070.

B. In einer mehrstufigen Reaktionsfolge z.B. ausgehend vom 4-Chlor-2-äthymercapto-pyrimidin-5-carbonsäureäthylester.

1. 2-Äthylmercapto-4-(N-2-äthoxycarbonyläthyl-N-cyclopropyl)-amino-pyrimidin-5-carbonsäureäthylester (VIII, R = Äthyl, X = Äthylmercapto, $R^3$ = H).

Eine Lösung von 123 g 4-Chlor-2-äthylmercapto-pyrimidin-5-carbonsäureäthylester in 400 ml Cyclohexan wird unter Eiskühlung und Rühren in rascher Tropfenfolge mit einem Gemisch von 78,5 g β-Cyclopropylamino-propionsäureäthylester und 50,5 g Triäthylamin bei ~10 °C versetzt. Man entfernt das Eisbad, wobei die Temperatur auf ~35 °C ansteigt. Dann läßt man über Nacht bei Raumtemperatur stehen, wäscht mit Wasser, trocknet mit $Na_2SO_4$ und destilliert das Lösungsmittel im Vakuum ab. Es werden 168,1 g der Titelverbindung als hellgelbes Öl erhalten. Der als Reaktant verwendete β-Cyclopropylaminopropionsäureäthylester wurde wie folgt hergestellt : Zu einer auf −60 bis −70 °C gekühlten Lösung von 57 g Cyclopropylamin in 150 ml Äthanol tropft man in ca. 3 Stunden 100 g auf −60 °C gekühlten, frisch destillierten Acrylsäureäthylester. Dann läßt man über Nacht langsam auf Raumtemperatur kommen, destilliert das Lösungsmittel im Vakuum ab und fraktioniert anschließend. Bei 104-110 °C/22 Torr gehen 122 g β-Cyclopropylamino-propionsäureäthylester über.

2. 2-Äthylmercapto-8-cyclopropyl-5-oxo-5,6,7,8-tetrahydropyrido [2,3-d] pyrimidin-6-carbonsäureäthylester (IX, R = Äthyl, X = Äthylmercapto, $R^3$ = H).

168,1 g roher 2-Äthylmercapto-4-(N-2-äthoxycarbonyläthyl-N-cyclopropyl)-amino-pyrimidin-5-carbonsäureäthylester werden in 600 ml wasserfreiem Toluol gelöst und unter Rühren rasch mit 61,5 g Kalium-tert.-butanolat versetzt. Man läßt über Nacht stehen, gibt 35 g Eisessig und 200 ml Wasser zu, trennt die Phasen, wäscht die Toluollösung nochmals mit Wasser, trocknet mit $Na_2SO_4$ und zieht das Toluol im Vakuum ab. Man erhält 168,1 g Carbonester. Eine Probe aus Cyclohexan umkristallisiert schmilzt bei 86-89 °C.

3. 2-Äthylmercapto-8-cyclopropyl-5-oxo-5,8-dihydro-pyrido [2,3-d] pyrimidin-6-carbonsäureäthylester (X, R = Äthyl, X = Äthylmercapto, $R^3$ = H).

168,1 g des nach 2) hergestellten Tetrahydro-pyridopyrimidin-6-carbonsäureäthylesters werden in 400 ml Chloroform gelöst und unter Eiskühlung bei ~10-15 °C rasch tropfenweise mit einer Lösung von 80 g Brom in 40 ml Chloroform versetzt. Dann wird noch 10 Minuten bei ~10 °C gerührt, mit 120 g Triäthylamin in ca. 10 Minuten versetzt und das Eisbad entfernt, wobei die Temperatur auf etwa 60-65 °C

steigt. Man rührt noch 1,5 Stunden nach, wäscht zweimal mit kaltem Wasser, trocknet mit Natriumsulfat, destilliert das Lösungsmittel im Vakuum ab und kristallisiert den Rückstand aus Isopropylalkohol um. Man erhält 156 g 2-Äthyl mercapto-8-cyclopropyl-5-oxo-5,8-dihydro-pyrido [2,3-d] pyrimidin-6-carbonsäureäthylester vom Schmelzpunkt 138 °C.

4. 2-Äthylmercapto-8-cyclopropyl-5-oxo-5,8-dihydro-pyrido [2,3-d] pyrimidin-6-carbonsäure (II, R = H, X = Äthylmercapto, $R^3$ = H)

110 g des nach 3) hergestellten Esters werden mit einer Lösung von 20 g Atzkali in 500 ml Wasser versetzt. Unter Rühren erhitzt man 30 Minuten auf 85-95 °C, filtriert die erhaltene Lösung bei Raumtemperatur und säuert mit 25 g Eisessig an. Der Niederschlag wird abgesaugt, mit Wasser gewaschen und im Vakuumtrockenschrank über Calciumchlorid getrocknet. Man erhält 85,3 g Pyrido [2,3-d] pyrimidin-6-carbonsäure vom Schmelzpunkt 228-230 °C, die für die weiteren Umsetzungen genügend rein ist. Eine Probe aus Äthanol umkristallisiert besitzt einen Schmelzpunkt von 235-236 °C.

Nach dem gleichen Verfahren wurde auch die entsprechende 2-Methylmercapto-8-cyclopropyl-5-oxo-5,8-dihydro-pyrido [2,3-d] pyrimidin-6-carbonsäure vom Schmelzpunkt 251 °C in vergleichbarer Ausbeute hergestellt.

## Beispiel 55

2-Piperidino-8-cyclopropyl-5-oxo-5,8-dihydro-pyrido [2,3-d] pyrimidin-6-carbonsäure (I, $R^1$ $R^2$ N = Piperidino, $R^3$ = H).

Eine Lösung von 29,3 g 2-Chlor-8-cyclopropyl-5-oxo-5,8-dihydro-pyrido [2,3-d] pyrimidin-6-carbonsäureäthylester in 150 ml wasserfreiem Toluol wird unter Eiskühlung und Rühren tropfenweise mit 18 g Piperidin (oder 8,6 g Piperidin und 11 g Triäthylamin) versetzt. Man erhitzt 1 Stunde unter Rückfluß zum Sieden, destilliert das Lösungsmittel im Vakuum ab, nimmt den Rückstand in Wasser auf, saugt den Niederschlag ab, suspendiert ihn in 50 ml Äthanol und versetzt mit einer Lösung von 6,2 g Ätzkali in 80 ml Wasser. Dann wird 2,5 Stunden rückfließend zum Sieden erhitzt, der Alkohol im Vakuum abdestilliert, mit ca. 100 ml $H_2O$ versetzt, filtriert und das Filtrat mit Eisessig angesäuert. Der Niederschlag wird abgesaugt, mit $H_2O$ gewaschen und im Vakuumtrockenschrank über $CaCl_2$ bei 80-100 °C getrocknet. Nach Umkristallisation aus Äthanol werden 26,7 g 2-Piperidino-8-cyclopropyl-5-oxo-5,8-dihydro-pyrido [2,3-d] pyrimidin-6-carbonsäure vom Schmelzpunkt 250-252 °C erhalten.

Nach diesem Verfahren können alle Verbindungen der Tabelle 1 mit Ausnahme des 2-Piperazinoderivats Nr. 7, hergestellt werden, weil Piperazin mit dem 2-Chlor-8-cyclopropyl-5-oxo-5,8-dihydro-pyrido [2,3-d] pyrimidin-6-carbonsäuremethylester zu einem Gemisch des Mono- und Disubstitutionsproduktes reagiert.

Der als Ausgangsmaterial verwendete 2-Chlor-8-cyclopropyl-5-oxo-5,8-dihydro-pyrido [2,3-d] pyrimidin-6-carbonsäureäthylester kann ausgehend vom 2,4-Dichlor-pyrimidin-5-carbonsäureäthylester in 3 Stufen hergestellt werden :

1. 2-Chlor-4-(N-2-äthoxycarbonyläthyl-N-cyclopropyl)-aminopyrimidin-5-carbonsäureäthylester (VIII, X = Chlor, R = Äthyl, $R^3$ = H).

Eine Lösung von 22,1 g 2,4-Dichlor-pyrimidin-5-carbonsäureäthylester in 150 ml wasserfreiem Cyclohexan wird unter Eiskühlung und Rühren bei ~ 10-15 °C tropfenweise mit einem Gemisch von 15,7 g β-Cyclopropylaminopropionsäureäthylester und 10,1 g Triäthylamin versetzt. Man rührt 1 Stunde bei 10-15 °C, läßt über Nacht bei Raumtemperatur stehen, wäscht mit Wasser, trocknet mit Natriumsulfat und destilliert das Lösungsmittel im Vakuum ab. Man erhält 34,1 g obigen Esters als nicht kristallisierendes hellgelbes Öl.

Entsprechend wurde die 6-Methylverbindung (VIII, X = Chlor, R = Äthyl, R = $CH_3$) ausgehend vom 2,4-Dichlor-6-methyl-pyrimidin-5-carbonsäureäthylester hergestellt.

2. 2-Chlor-8-cyclopropyl-5-oxo-5,6,7,8-tetrahydro-pyrido [2,3-d] pyrimidin-6-carbonsäureäthylester (IX, R = Äthyl, X = Chlor, $R^3$ = H).

Eine Lösung von 34,1 g 2-Chlor-4-(N-2-äthoxycarbonyläthyl-N-cyclopropyl)-amino-pyrimidin-5-carbonsäureäthylester in 250 ml wasserfreiem Toluol wird unter Eiskühlung und Rühren portionsweise mit 11,2 g Kalium-tert.-butanolat versetzt. Man rührt 5 Stunden bei Raumtemperatur, gibt 150 ml Eiswasser zu, säuert mit 4n HCl an, trennt die Phasen, trocknet mit $Na_2SO_4$ und zieht das Lösungsmittel im Vakuum ab. Es werden 25 g Rohprodukt erhalten, das direkt in die nächste Reaktionsstufe eingesetzt wird.

Der entsprechende 4-Methyl-pyrido [2,3-d] pyrimidin-carbonsäureäthylester (IX, R = Äthyl, X = Chlor, $R^3$ = $CH_3$) schmilzt nach Umkristallisation aus Toluol/Leichtbenzin bei 110-112 °C.

3. 2-Chlor-8-cyclopropyl-5-oxo-5,8-dihydro-pyrido [2,3-d] pyrimidin-6-carbonsäureäthylester (X, R = Äthyl, X = Chlor, $R^3$ = H).

25 g der zuvor beschriebenen Verbindung werden in 100 ml Chloroform gelöst und unter Eiskühlung und Rühren tropfenweise mit einer Lösung von 11,7 g Brom in 40 ml Chloroform bei 0-10 °C versetzt. Man rührt 30 Minuten bei ~ 10 °C, tropft bei 10-15 °C 15 g Triäthylamin langsam zu, rührt 1 Stunde unter Eiskühlung weiter und dann anschließend 6 Stunden bei Raumtemperatur. Man wäscht mit Eiswasser, trocknet mit Natriumsulfat und zieht das Lösungsmittel im Vakuum ab. Nach Umkristallisation des

Rückstandes aus Toluol/Cyclohexan werden 16 g 2-Chlor-8-cyclopropyl-5-oxo-5,8-dihydropyrido [2,3-d] pyrimidin-6-carbonsäureäthylester vom Schmelzpunkt 169 °C erhalten.

Beispiel 56

2-Pyrrolidino-8-cyclopropyl-5-oxo-5,8-dihydro-pyrido [2,3-d] pyrimidin-6-carbonsäure (I, $R^1$ $R^2$ N = Pyrrolidino, $R^3$ = H).

Eine Lösung von 17,1 g 2-Chlor-4-(N-2-äthoxycarbonyläthyl-N-cyclopropyl)-amino-pyrimidin-5-carbonsäureäthylester in 100 ml wasserfreiem Toluol wird tropfenweise mit 7,8 g Pyrrolidin versetzt, wobei die Temperatur der Lösung auf ~ 40-50 °C steigt. Man rührt 30 Minuten, kocht 30 Minuten rückfließend, wäscht die erkaltete Lösung mit $H_2O$, trocknet mit $Na_2SO_4$ und destilliert das Lösungsmittel im Vakuum ab und erhält 18,5 g rohen 2-Pyrrolidino-4-(N-2-äthoxycarbonyläthyl-N-cyclopropyl)-amino-pyrimidin-5-carbonsäureäthylester, den man in 120 ml Toluol löst und rasch portionsweise mit 6,5 g Kalium-tert.-butanolat versetzt. Man läßt über Nacht bei Raumtemperatur stehen, gibt 100 ml Wasser und 3,5 g Eisessig zu, trennt die Toluolphase ab, wäscht mit $H_2O$, trocknet über $Na_2SO_4$ und zieht das Lösungsmittel im Vakuum ab. Nach Umkristallisation aus Cyclohexan erhält man 12,5 g 2-Pyrrolidino-8-cyclopropyl-5-oxo-5,6,7,8-tetrahydro-pyrido [2,3-d] pyrimidin-6-carbonsäureäthylester vom Schmelzpunkt 72-74 °C, die man in 150 ml Chloroform löst und unter Eiskühlung und Rühren tropfenweise mit einer Lösung von 6,2 g Brom in 20 ml $CHCl_3$ versetzt. Man rührt 2 Stdn. bei Raumtemperatur, versetzt unter Eiskühlung und Rühren tropfenweise mit 8,5 g Triäthylamin, rührt 1 Stde. bei Raum = temperatur sowie 1 Stde. bei 50 °C, wäscht mit Wasser, trocknet über $Na_2SO_4$ und destilliert das Lösungsmittel i. Vak. ab. Man erhält 11,5 g 2-Pyrrolidino-8-cyclopropyl-5-oxo-5,8-dihydro-pyrido [2,3-d] pyrimidin-6-carbonsäureäthylester den man zur Verseifung mit 50 ml Äthanol und einer Lösung von 5 g Ätzkali in 100 ml Wasser versetzt. Man erhitzt 2 Stdn. unter Rückfluß zum Sieden, destilliert den Alkohol möglichst vollständig i. Vak ab, bringt das carbonsäure Salz mit $H_2O$ in Lösung, filtriert und säuert mit Eisessig auf pH = 5 an. Der Niederschlag wird abgesaugt, mit $H_2O$ gewaschen und im Vakuumtrocken = schrank über $CaCl_2$ bei 80-100 °C getrocknet. Nach Umkristallisation aus Dimethylformamid/Äthanol werden 9,2 g 2-Pyrrolidino-8-cyclopropyl-5-oxo-5,8-dihydro-pyrido-[2,3-d]-pyrimidin-6-carbonsäure vom Zersp. 306 °C erhalten. (Nr. 4 der Tabelle 1).

Nach dem gleichen Verfahren wurden auch die 2-Piperidino- sowie die 2-Morpholino-8-cyclopropyl-5-oxo-5,8-dihydropyrido [2,3-d] pyrimidin-6-carbonsäure hergestellt (Nr. 6 und 5 der Tabelle 1).

Beispiel 57

2-Pyrrolidino-4-methyl-8-cyclopropyl-5-oxo-5,8-dihydro-pyrido [2,3-d] pyrimidin-6-carbonsäure (I, $R^1$ $R^2$ N = Pyrrolidino, $R^3$ = $CH_3$).

3,4 g 2-Pyrrolidino-4-methyl-8-cyclopropyl-5-oxo-5,6,7,8-tetra = hydro-pyrido [2,3-d] pyrimidin-6-carbonsäureäthylester werden mit 100 ml Toluol und 2,5 g Chloranil versetzt. Man erwärmt 30 Min. auf 80-90 °C, zieht das Lösungsmittel i. Vak. ab und kristallisiert den Rückstand aus Acetonitril um. Die erhaltenen 2,2 g des Esters vom Schmelzpunkt 170-175° werden mit 20 ml Äthanol und einer Lösung von 1,2 g Ätzkali in 60 ml $H_2O$ 2,5 Stdn. rückfließend zum Sieden erhitzt. Der Alkohol wird abdestilliert, die filtrierte Lösung mit Eisessig eingesäuert und der Niederschlag abgesaugt, mit Wasser gewaschen und getrocknet. Man erhält 1,6 g 2-Pyrrolidino-4-methyl-8-cyclopropyl-5-oxo-5,8-dihydro-pyrido [2,3-d] pyrimidin-6-carbonsäure vom Schmelzpunkt 253 °C. (Nr. 2 der Tabelle 1).

Nach dem gleichen Verfahren wurde auch die in der Tabelle 1 als Nr. 3 erwähnte Verbindung hergestellt.

Der als Ausgangsmaterial verwendete 2-Pyrrolidino-4-methyl-8-cyclopropyl-5-oxo-5,6,7,8-tetrahydro-pyrido [2,3-d] pyrimidin-6-carbonsäure-äthylester wird wie folgt hergestellt: 31 g 2-Chlor-4-methyl-8-cyclopropyl-5-oxo-5,6,7,8-tetrahydro-pyrido [2,3-d] pyrimidin-6-carbonsäureäthylester werden in 300 ml Toluol gelöst und rasch tropfenweise mit 20 g Pyrrolidin versetzt wobei die Temperatur auf ca 40 °C ansteigt. Man erhitzt 30 Minuten unter Rückfluß zum Sieden, wäscht mit Wasser, trocknet mit $Na_2SO_4$ und zieht das Lösungsmittel im Vakuum ab. Der Rückstand wird aus Waschbenzin (Kp 110-140 °C) umkristallisiert. Man erhält 30 g farbslose Kristalle vom Schmelzpunkt 121-123 °C.

Es wurde weiterhin gefunden, daß die erfindungsgemäßen neuen Verbindungen hervorragende antimikrobielle Eigenschaften besitzen und darüber hinaus als Wachstumsregulatoren wirksam sind.

Insbesondere sind sie breit bakteriostatisch und bakterizid gegen grampositive Bakterien, wie Staphylokokken und Streptokokken, und gramnegative Bakterien wie Escherichia, Proteus, Providencia, Enterobacter, Klebsiella und Pseudomonas, wirksam. Diese Aufzählung von Erregern ist lediglich beispielhaft und nicht beschränkend aufzufassen. Die verbesserte antibakterielle Wirkung der erfindungsgemäßen neuen Verbindungen wird besonders deutlich an den Beispielen 1 und 7, die sich im Vergleich zu 2-Piperazino-8-ethyl-5-oxo-5,8-dihydro-pyrido [2,3-d] pyrimidin-6-carbonsäure (« Pipemidin Säure ») oder der bekannten 1-Äthyl-7-methyl-4-naphthyridon-(1,8)-3-carbonsäure (« Nalidixinsäure » ; Ehrhart/Ruschig, Arzneimittel, Band 2 : Chemotherapeutika, Verlag Chemie 1968, Seite 1568) in vitro

bzw. in vivo bei Escherichia coli, Proteus, Klebsiella, Pseudomonas usw. als weit überlegen erwiesen.

Die verbesserte antibakterielle Wirksamkeit der erfindungsgemäßen Verbindungen ermöglicht ihren Einsatz als Wirkstoffe sowohl in der Human- als auch in der Veterinärmedizin, wobei sie sowohl zur Verhütung als auch zur Behandlung von systemischen oder lokalen bakteriellen Infektionen, insbesondere von Erkrankungen des Urogenitalsystems verwendet werden können. Die erfindungsgemäßen Verbindungen können weiterhin auch als Futterzusatzmittel zur Förderung des Wachstums und zur Verbesserung der Futterauswertung in der Tierhaltung, insbesondere bei der Haltung von Mastvieh, verwendet werden. Die Applikation der Wirkstoffe erfolgt dann vorzugsweise über das Futter und/oder das Trinkwasser.

Die vorliegende Erfindung betrifft weiterhin Mittel, die die neuen erfindungsgemäßen Verbindungen enthalten. Hierzu gehören beispielsweise Futtermittelkonzentrate für die Tierhaltung, die in üblicher Weise neben den Wirkstoffen auch Vitamine und/oder Mineralsalze enthalten können oder pharmazeutische Zubereitungen.

Die Erfindung betrifft bevorzugt antibakteriell wirksame Mittel, die Verbindungen der Formel (I) enthalten. Die Erfindung betrifft besonders bevorzugt solche antibakteriell wirksamen Mittel, die Verbindungen der Formel oder deren Alkali- oder Erdalkalisalze enthalten.

Die erfindungsgemäßen pharmazeutischen Zubereitungen enthalten neben den neuen erfindungsgemäßen Verbindungen in üblicher Weise nicht toxische, inerte pharmazeutisch geeignete Trägerstoffe. Solche pharmazeutisch geeignete Trägerstoffe sind beispielsweise Füll- und Streckmittel, Bindemittel, Feuchthaltemittel, Lösungsverzögerer, Resorptionsbeschleuniger, Netzmittel, Adsorbtionsmittel oder Gleitmittel, die feste, halbfeste oder flüssige Konsistenz haben können. Solche pharmazeutisch geeignete Trägerstoffe sind dem Fachmann bekannt.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotionen, Puder und Sprays genannt. Die Herstellung dieser Zubereitungen geschieht nach bekannten Methoden in üblicher Weise, beispielsweise durch Mischen des neuen erfindungsgemäßen Wirkstoffes mit den üblichen Träger- und Zusatzstoffen. Der Wirkstoff soll in den aufgeführten pharmazeutischen Zubereitungen in einer Konzentration von etwa 0,1 bis 99,5, vorzugsweise von etwa 0,5 bis 95 Gew.-% der Gesamtmischung vorhanden sein.

Die Bereitstellung neuer Bakterizide zur Bekämpfung von Bakterien, die gegen bekannte Bakterizide resistent sind, ist eine Bereicherung des Standes der Technik.

**Ansprüche**

1. 2-Amino-8-cyclopropyl-5-oxo-5,8-dihydro-pyrido-[2,3-d] pyrimidin-6-carbonsäuren der allgemeinen Formel I

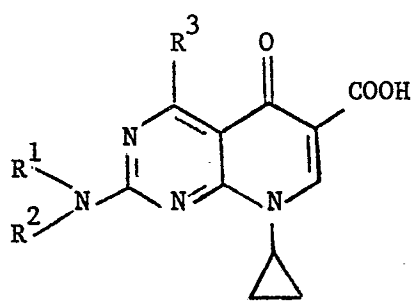

(I)

in welcher

$R^1$ und $R^2$ gleich oder verschieden sein können und für Wasserstoff, einen verzweigten oder unverzweigten Alkylrest mit 1-12 Kohlenstoffatomen, einen Alkenyl- oder Alkinylrest mit 3-12 Kohlenstoffatomen stehen, wobei sich die Doppel- bzw. Dreifachbindung nicht an dem mit dem Stickstoffatom verbundenen Kohlenstoffatom befinden darf, und der gegebenenfalls durch Hydroxylgruppen, Alkoxy-, Alkylmercapto- oder Dialkylamino-gruppen mit 1-3 Kohlenstoffatomen für einen Alkylrest, die Nitril-, Alkoxycarbonyl-gruppe mit 1-4 Kohlenstoffatomen im Alkoxyteil sowie einen Aryl- oder Hetarylrest substituiert sein kann, ferner Cycloalkyl mit 3-6 Kohlenstoffatomen bedeutet und weiterhin gemeinsam mit dem Stickstoffatom, das sie substituieren und gegebenenfalls Sauerstoff, Schwefel oder $NR^4$ einen 3-7-gliedrigen Ring bilden, der ein- oder mehrfach durch einen Alkylrest mit 1-6 Kohlenstoffatomen oder Alkenyl-gruppen mit 3-6 Kohlenstoffatomen, wobei sich die Doppelbindung nicht an dem mit dem Stickstoffatom verbundenen Kohlenstoffatom befinden darf, Hydroxyl-, Alkoxy- und Alkylmercaptogruppen mit 1-3 Kohlenstoffatomen, eine Alkoxycarbonyl-gruppe mit 1-4 Kohlenstoffatomen im Alkoholteil, die Nitrilgruppe sowie einen Arylrest substituiert sein kann und ferner eine Doppelbindung besitzen kann,

$R^3$ für Wasserstoff oder Alkyl mit bis zu 3 Kohlenstoffatomen steht und

$R^4$ Wasserstoff, eine verzweigte oder unverzweigte Alkyl-, Alkenyl- oder Alkinyl-gruppe mit 1-6 Kohlenstoffatomen, die gegebenenfalls durch Hydroxyl, Alkoxy- und Alkylmercapto- oder Dialkylaminogruppe mit 1-3 Kohlenstoffatomen für einen Alkylrest, die Alkoxycarbonylgruppe mit 1-4 Kohlenstoffatomen im Alkoxyteil substituiert sein kann, eine Cycloalkylgruppe mit 3-7 Kohlenstoffatomen, eine gegebenenfalls im Arylrest durch Halogen, Alkyl-, Alkoxy- oder Alkylmercaptogruppen mit 1-3 Kohlenstoffatomen, Aryloxy-, Arylmercapto- sowie eine Trifluormethyl-, Nitro-, Nitril- oder Alkoxycarbonylgruppe mit 1 — 4 Kohlenstoffatomen im Alkoxyteil ein- oder mehrfach substituierte Aralkylgruppe mit bis zu 4 Kohlenstoffatomen im aliphatischen Teil, sowie eine gegebenenfalls durch Halogen, Alkyl, Alkoxy oder Alkylmercapto-gruppen mit 1-3 Kohlenstoffatomen, eine Trifluormethyl-, Nitro- oder Nitrilgruppe substituierte Phenyl- oder Naphthylgruppe oder ein heterocyclischer Rest wie beispielsweise einen Pyridin-, Pyrimidin-, Thiazol- oder Benzthiazolkern darstellt, ferner eine gegebenenfalls durch einen Arylrest substituierte Alkoxycarbonylgruppe mit 1-4 Kohlenstoffatomen im Alkoxyteil, ein Alkanoylrest mit 1-6 Kohlenstoffatomen, ein Aroylrest, ein Alkylthiocarbamoylrest mit 1-4 Kohlenstoffatomen im Alkylteil oder ein Arylcarbamoylrest oder ein gegebenenfalls durch Halogen, Alkyl, Alkoxy oder Alkylmercaptogruppen mit 1-3 Kohlenstoffatomen, eine Trifluormethyl-, Nitro- oder Nitrilgruppe substituierter Aryl-(thio)carbamoylrest, ein Alkyl- oder Arylsulfonylrest sowie ein gegebenenfalls durch Alkyl substituierter Sulfamoylrest bedeutet, sowie deren pharmazeutisch verwertbaren Salze.

2. 2-(4-Methyl-piperazino)-8-cyclopropyl-5-oxo-5,8-dihydro-pyrido [2,4-d] pyrimidin-6-carbonsäure.

3. 2-Pyrrolidino-8-cyclopropyl-5-oxo-5,8-dihydro-pyrido-[2,3-d] pyrimidin-6-carbonsäure.

4. 2-Piperazino-8-cyclopropyl-5-oxo-5,8-dihydro-pyrido [2,3-d]-pyrimidin-6-carbonsäure.

5. 2-(4-ß-Hydroxyäthyl-piperazino)-8-cyclopropyl-5-oxo-5,8-dihydro-pyrido [2,3-d] pyrimidin-6-carbonsäure.

6. 2-Dimethylamino-8-cyclopropyl-5-oxo-5,8-dihydro-pyrido-[2,3-d] pyrimidin-6-carbonsäure.

7. Verfahren zur Herstellung von 2-Amino-8-cyclopropyl-5-oxo-5,8-dihydro-pyrido [2,3-d] pyrimidin-6-carbonsäuren gemäß Anspruch 1, dadurch gekennzeichnet, daß man

a) Pyrido [2,3-d] pyrimidin-6-carbonsäuren der allgemeinen Formel II

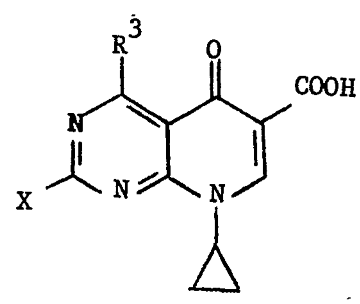

(II)

in welcher

$R^3$ die oben angegebene Bedeutung hat und

X für ein Halogenatom oder eine Alkylmercapto- oder Alkoxygruppe mit 1-4 Kohlenstoffatomen steht, mit Aminen der Formel III

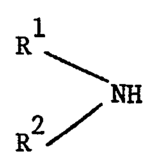

(III)

in welcher

$R^1$ und $R^2$ die oben angegebene Bedeutung haben, umsetzt, oder

b) Pyrido [2,3-d] pyrimidin-6-carbonsäureester der allgemeinen Formel IIa

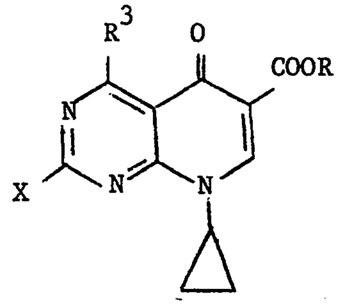

(IIa)

in welcher

$R^3$ die oben angegebene Bedeutung hat,

R eine Alkylgruppe und

X ein Halogenatom bedeutet, in Gegenwart eines Säurebinders mit den Aminen der Formel III umsetzt und anschließend den Ester alkalisch verseift, oder

c) 2-Halogen-4-(N-2-alkoxycarbonylalkyl-N-cyclopropyl)-amino-pyrimidin-5-carbonsäureester mit den Aminen der Formel III umsetzt, die dabei entstehenden Verbindungen der Cyclisierung unterwirft und die erhaltenen Produkte dehydriert und verseift oder

d) 2-Halogen-8-cyclopropyl-5-oxo-5,6,7,8-tetrahydro-pyrido [2,3-d] pyrimidin-6-carbonsäureester mit den Aminen der Formel III umsetzt, die entstehenden 2-Amino-8-cyclopropyl-5-oxo-5,6,7,8-tetrahydro-pyrido [2,3-d] pyrimidin-6-carbonsäureester dehydriert und verseift.

8. Arzneimittel, gekennzeichnet durch einen Gehalt an mindestens einer 2-Amino-8-cyclopropyl-5-oxo-5,8-dihydro-pyrido [2,3-d] pyrimidin-6-carbonsäure gemäß Anspruch 1.

9. Verfahren zur Herstellung von antibakteriellen Mitteln, dadurch gekennzeichnet, daß man 2-Amino-8-cyclopropyl-5-oxo-5,8-dihydro-pyrido [2,3-d] pyrimidin-6-carbonsäuren gemäß Anspruch 1 mit inerten, nichttoxischen, pharmazeutisch geeigneten Trägerstoffen vermischt.

## Claims

1. 2-Amino-8-cyclopropyl-5-oxo-5,8-dihydro-pyrido-[2,3-d]-pyrimidine-6-carboxylic acids of the general formula I

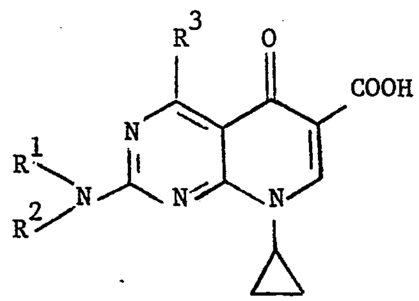

(I)

in which

$R^1$ and $R^2$ can be identical or different and represent hydrogen, a branched or unbranched alkyl radical with 1-12 carbon atoms, an alkenyl or alkinyl radical with 3-12 carbon atoms, in which the double or triple bond may not be on the carbon atom linked to the nitrogen atom, and which can optionally be substituted by hydroxyl groups or alkoxy, alkylmercapto or dialkylamino groups with 1-3 carbon atoms per alkyl radical, the nitrile group, the alkoxycarbonyl group with 1-4 carbon atoms in the alkoxy part as well as an aryl or hetaryl radical, or also denote cycloalkyl with 3-6 carbon atoms and, furthermore, together with the nitrogen atom on which they are substituents and optionally with oxygen, sulphur or $NR^4$, form a 3-membered to 7-membered ring, which can be monosubstituted or polysubstituted by an alkyl radical with 1-6 carbon atoms or alkenyl groups with 3-6 carbon atoms, in which the double bond may not be on the carbon atom linked to the nitrogen atom, hydroxyl groups, alkoxy and alkylmercapto groups with 1-3 carbon atoms, an alkoxycarbonyl group with 1-4 carbon atoms in the alcohol part, the nitrile group or an aryl radical and also can possess a double bond,

$R^3$ represents hydrogen or alkyl with up to 3 carbon atoms and

$R^4$ represents hydrogen, a branched or unbranched alkyl, alkenyl or alkinyl group with 1-6 carbon atoms, which optionally can be substituted by hydroxyl, an alkoxy, alkylmercapto or dialkylamino group with 1-3 carbon atoms per alkyl radical or the alkoxycarbonyl group with 1-4 carbon atoms in the alkoxy part, or represents a cycloalkyl group with 3-7 carbon atoms, an aralkyl group which has up to 4 carbon atoms in the aliphatic part and is optionally monosubstituted or polysubstituted in the aryl radical by halogen, alkyl, alkoxy or alkylmercapto groups with 1-3 carbon atoms, aryloxy, arylmercapto or a trifluoromethyl, nitro, nitrile or alkoxycarbonyl group with 1-4 carbon atoms in the alkoxy part, or also a phenyl or naphthyl group optionally substituted by halogen, alkyl, alkoxy or alkylmercapto groups with 1-3 carbon atoms, a trifluoromethyl, nitro or nitrile group, or represents a heterocyclic radical such as, for example, a pyridine, pyrimidine, thiazole or benzthiazole nucleus, or also denotes an alkoxycarbonyl group which has 1-4 carbon atoms in the alkoxy part and is optionally substituted by an aryl radical, or denotes an alkanoyl radical with 1-6 carbon atoms, an aroyl radical, an alkylthiocarbamoyl radical with 1-4 carbon atoms in the alkyl part or an arylcarbamoyl radical or an aryl-(thio)carbamoyl radical optionally substituted by halogen, alkyl, alkoxy or alkylmercapto groups with 1-3 carbon atoms, a trifluoromethyl, nitro or nitrile group, or denotes an alkyl or arylsulphonyl radical or an optionally alkyl-substituted sulphamoyl radical, and their pharmaceutically usable salts.

2. 2-(4-Methyl-piperazino)-8-cyclopropyl-5-oxo-5,8-dihydropyrido [2,4-d] pyrimidine-6-carboxylic acid.

3. 2-Pyrrolidino-8-cyclopropyl-5-oxo-5,8-dihydro-pyrido-[2,3-d] pyrimidine-6-carboxylic acid.

4. 2-Piperazino-8-cyclopropyl-5-oxo-5,8-dihydro-pyrido-[2,3-d]-pyrimidine-6-carboxylic acid.

5. 2-(4-ß-Hydroxyethyl-piperazino)-8-cyclopropyl-5-oxo-5,8-dihydro-pyrido [2,3-d] pyrimidine-6-carboxylic acid.

6. 2-Dimethylamino-8-cyclopropyl-5-oxo-5,8-dihydro-pyrido-[2,3-d] pyrimidine-6-carboxylic acid.

7. Process for the preparation of 2-amino-8-cyclopropyl-5-oxo-5,8-dihydro-pyrido [2,3-d] pyrimidine-6-carboxylic acids according to Claim 1, characterised in that

a) pyrido [2,3-d] pyrimidine-6-carboxylic acids of the general formula II

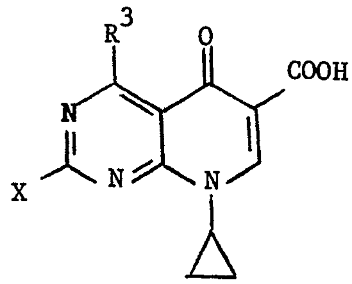

(II)

in which

$R^3$ has the abovementioned meaning and

X represents a halogen atom or an alkylmercapto or alkoxy group with 1-4 carbon atoms, are reacted with amines of the formula III

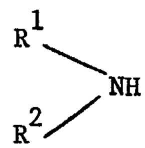

(III)

in which

$R^1$ and $R^2$ have the abovementioned meaning, or

b) pyrido [2,3-d] pyrimidine-6-carboxylic acid esters of the general formula IIa

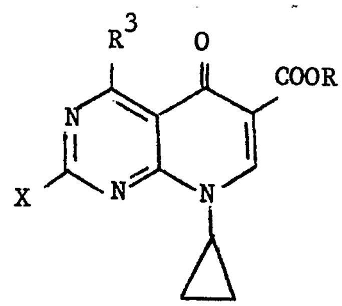

(IIa)

in which

$R^3$ has the abovementioned meaning,

R denotes an alkyl group and

X denotes a halogen atom, are reacted in the presence of an acid-binding agent with the amines of the formula III and the ester is then subjected to alkaline saponification, or

c) 2-halogeno-4-(N-2-alkoxycarbonylalkyl-N-cyclopropyl)-amino-pyrimidine-5-carboxylic acid esters are reacted with the amines of the formula III, the compounds thus formed are subjected to cyclisation and the resulting products are dehydrogenated and saponified, or

d) 2-halogeno-8-cyclopropyl-5-oxo-5,6,7,8-tetrahydro-pyrido [2,3-d] pyrimidine-6-carboxylic acid esters are reacted with the amines of the formula III and the resulting 2-amino-8-cyclopropyl-5-oxo-5,6,7,8-tetrahydro-pyrido [2,3-d] pyrimidine-6-carboxylic acid esters are dehydrogenated and saponified.

8. Medicament, characterised in that it contains at least one 2-amino-8-cyclopropyl-5-oxo-5,8-dihydro-pyrido [2,3-d] pyrimidine-6-carboxylic acid according to Claim 1.

9. Process for the preparation of antibacterial agents, characterised in that 2-amino-8-cyclopropyl-5-oxo-5,8-dihydro-pyrido [2,3-d] pyrimidine-6-carboxylic acids according to Claim 1 are mixed with inert, non-toxic, pharmaceutically suitable excipients.

**Revendications**

1. Acides 2-amino-8-cyclopropyl-5-oxo-5,8-dihydro-pyrido-[2,3-d] pyrimidine-6-carboxylique de formule générale I

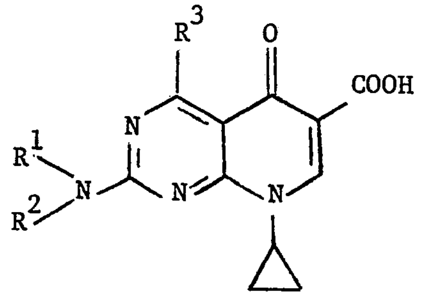

(I)

dans laquelle

$R^1$ et $R^2$ peuvent être identiques ou différents et représentent l'hydrogène, un reste alkyle droit ou ramifié en $C_1$-$C_{12}$, un reste alcényle ou alcynyle en $C_3$-$C_{12}$, étant précisé que la double liaison ou triple liaison ne peut se trouver sur l'atome de carbone relié à l'atome d'azote, et qui peut être éventuellement substitué par des groupes hydroxy, des groupes alcoxy, alkylmercapto ou dialkylamino contenant 1 à 3 atomes de carbone par groupe alkyle, le groupe nitrile, le groupe alcoxycarbonyle contenant 1 à 4 atomes de carbone dans la partie alcoxy ou un reste aryle ou hétéroaryle, ou représente un groupe cycloalkyle en $C_3$-$C_6$, et en outre peuvent former avec l'atome d'azote dont ils sont substituants et le cas échéant l'oxygène, le soufre ou $NR^4$, un cycle de 3 à 7 chaînons qui peut être mono- ou poly-substitué par un reste alkyle en $C_1$-$C_6$ ou des groupes alcényle en $C_3$-$C_6$, étant précisé que la double liaison ne peut se trouver sur l'atome de carbone relié à l'atome d'azote, des groupes hydroxy, alcoxy ou alkylmercapto en $C_1$-$C_3$, un groupe alcoxycarbonyle contenant 1 à 4 atomes de carbone dans la partie alcool, le groupe nitrile ou un reste aryle, et peut en outre contenir une double liaison,

$R^3$ représente l'hydrogène ou un groupe alkyle contenant jusqu'à 3 atomes de carbone, et

$R^4$ représente l'hydrogène, un groupe alkyle, alcényle ou alcynyle en $C_1$-$C_6$ droit ou ramifié, qui peut être éventuellement substitué par des groupes hydroxy, alcoxy et alkylmercapto ou dialkylamino contenant 1 à 3 atomes de carbone par groupe alkyle, le groupe alcoxycarbonyle contenant 1 à 4 atomes de carbone dans la partie alcoxy, ou encore un groupe cycloalkyle en $C_3$-$C_7$, un groupe aralkyle contenant jusqu'à 4 atomes de carbone dans la partie aliphatique et qui est éventuellement mono- ou poly-substitué dans le reste aryle par des halogènes, des groupes alkyle, alcoxy ou alkylmercapto en $C_1$-$C_3$, aryloxy, arylmercapto ou un groupe trifluorométhyle, nitro, nitrile ou un groupe alcoxycarbonyle contenant 1 à 4 atomes de carbone dans la partie alcoxy, ou encore un groupe phényle ou naphtyle éventuellement substitué par des halogènes, des groupes alkyle, alcoxy ou alkylmercapto en $C_1$-$C_3$, un groupe trifluorométhyle, nitro ou nitrile ou encore un reste hétérocyclique, par exemple un noyau de pyridine, de pyrimidine, de thiazole ou de benzothiazole, ou encore un groupe alcoxycarbonyle contenant 1 à 4 atomes de carbone dans la partie alcoxy et éventuellement substitué par un reste aryle, un reste alcanoyle en $C_1$-$C_6$, un reste aroyle, un reste alkylthiocarbamoyle contenant 1 à 4 atomes de carbone dans la partie alkyle ou un reste arylcarbamoyle ou un reste aryl-(thio)-carbamoyle éventuellement substitué par des halogènes, des groupes alkyle, alcoxy ou alkylmercapto en $C_1$-$C_3$, un groupe trifluorométhyle, nitro ou nitrile, un reste alkyl- ou aryl-sulfonyle ou encore un reste sulfamoyle portant éventuellement des substituants alkyle, et leurs sels acceptables pour l'usage pharmaceutique.

2. L'acide 2-(4-méthyl-pipérazino)-8-cyclopropyl-5-oxo-5,8-dihydro-pyrido [2,4-d] pyrimidine-6-carboxylique.

3. L'acide 2-pyrrolidino-8-cyclopropyl-5-oxo-5,8-dihydropyrido [2,3-d] pyrimidine-6-carboxylique.

4. L'acide 2-pipérazino-8-cyclopropyl-5-oxo-5,8-dihydropyrido [2,3-d] pyrimidine-6-carboxylique.

5. L'acide 2-(4-ß-hydroxyéthyl-pipérazino)-8-cyclopropyl-5-oxo-5,8-dihydro-pyrido [2,3-d] pyrimidine-6-carboxylique.

6. L'acide 2-diméthylamino-8-cyclopropyl-5-oxo-5,8-dihydro-pyrido [2,3-d] pyrimidine-6-carboxylique.

7. Procédé de préparation des acides 2-amino-8-cyclopropyl-5-oxo-5,8-dihydro-pyrido [2,3-d] pyrimidine-6-carboxylique selon la revendication 1, caractérisé en ce que :

a) on fait réagir des acides pyrido [2,3-d] pyrimidine-6-carboxyliques de formule générale II

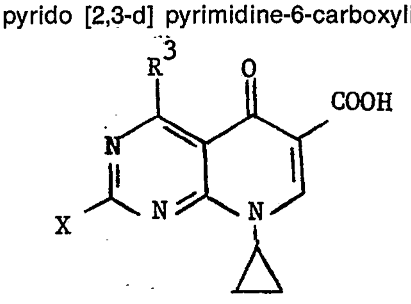

(II)

dans laquelle

$R^3$ a la signification indiquée ci-dessus et

X représente un atome d'halogène ou un groupe alkylmercapto ou alcoxy en $C_1$-$C_4$, avec des amines de formule III

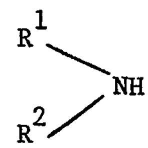

(III)

dans laquelle

$R^1$ et $R^2$ ont les significations indiquées ci-dessus, ou bien

b) on fait réagir les esters d'acides pyrido [2,3-d] pyrimidine-6-carboxyliques de formule générale IIa

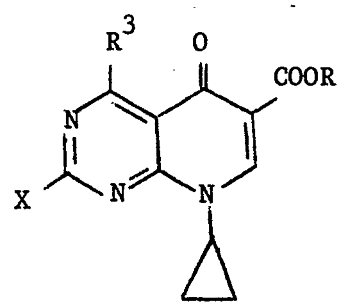

(IIa)

dans laquelle

$R^3$ a la signification indiquée ci-dessus,

R représente un groupe alkyle et

X un atome d'halogène, en présence d'un agent fixant les acides, avec les amines de formule III, et on soumet ensuite les esters à saponification alcaline, ou bien

c) on fait réagir des esters d'acides 2-halogéno-4-(N-2-alcoxy-carbonylalkyl-N-cyclopropyl)-amino-pyrimidine-5-carboxyliques avec les amines de formule III, on soumet les composés obtenus à cyclisation et on soumet les produits obtenus à déshydrogénation et saponification, ou bien

d) on fait réagir des esters d'acides 2-halogéno-8-cyclopropyl-5-oxo-5,6,7,8-tétrahydro-pyrido [2,3-d] pyrimidine-6-carboxyliques avec les amines de formule III, ce qui donne des esters d'acides 2-amino-8-cyclopropyl-5-oxo-5,6,7,8-tétrahydro-pyrido [2,3-d] pyrimidine-6-carboxyliques qu'on soumet à déshydrogénation et saponification.

8. Médicament caractérisé en ce qu'il contient au moins un acide 2-amino-8-cyclopropyl-5-oxo-5,8-dihydro-pyrido [2,3-d] pyrimidine-6-carboxylique selon la revendication 1.

9. Procédé de préparation d'agents antibactériens caractérisé en ce que l'on mélange des acides 2-amino-8-cyclopropyl-5-oxo-5,8-dihydro-pyrido [2,3-d] pyrimidine-6-carboxyliques selon la revendication 1 avec des véhicules inertes non toxiques appropriés à l'usage pharmaceutique.